# EUROPEAN PATENT SPECIFICATION

(11) **EP 4 185 561 B1**
(45) Date of publication and mention of the grant of the patent: **03.09.2025**
(21) Application number: 21742531.3
(22) Date of filing: 16.07.2021
(51) Int. Cl.: C07C 41/06, C07C 41/42, C07C 43/04

(54) **METHOD FOR PRODUCTION OF MTBE**
VERFAHREN ZUR HERSTELLUNG VON MTBE
PROCÉDÉ DE PRODUCTION DE MTBE

(30) Priority: 21.07.2020 EP 20187063
(43) Date of publication of application: 31.05.2023
(73) Proprietor: SABIC Global Technologies B.V., 4612 PX Bergen op Zoom (NL)
(72) Inventor: ANSARI, Mohammed Bismillah, Riyadh 11551 (SA); LEAL CANELON, Guillermo, Riyadh 11551 (SA); BODAS, Vijay Dinkar, Riyadh 11551 (SA); HASYAGAR, Umesh, Bangalore 562125 (IN); NAIR, Vinod, Bangalore 562125 (IN)
(74) Representative: EP&C
(86) International application number: PCT/IB2021/056472
(87) International publication number: WO 2022/018600

(56) References cited:
- EP-A2- 0 366 501
- US-A1- 2006 065 574

## Description

### CROSS REFERENCE TO RELATED APPLICATIONS

This application claims the benefit of priority to European Patent Application No. 20187063.1, filed July 21, 2020.

### FIELD OF INVENTION

The present invention generally relates to methods for producing methyl tert-butyl ether (MTBE). More specifically, the present invention relates to a method for producing MTBE using a reaction unit that includes two parallel MTBE synthesis reactors that are in series with a third MTBE synthesis reactor. US 2006/065574 discloses a MTBE process with three reactors in series.

### BACKGROUND OF THE INVENTION

MTBE is an organic compound that is used as an additive to enhance the octane number of gasoline. Since about 1970, MTBE has been synthesized by etherification of isobutylene by reaction with methanol in the presence of an acidic catalyst. Isobutylene used for MTBE synthesis can be obtained from C₄ hydrocarbon process streams.

Conventionally, isobutylene and methanol are fed into a fixed bed reactor to produce an MTBE containing effluent. The effluent is then fed to a catalytic distillation column or a reactive distillation column to react isobutylene remaining in the effluent with additional methanol to produce more MTBE. Some other conventional processes also use super fractionator for separation of light ends (C₄ and methanol) from MTBE. The catalytic distillation column and/or super fractionator generally require a large amount of capital expenditure and operational costs, thereby increasing the production costs for MTBE. Other MTBE production systems use isothermal multi-tubular reactors as the MTBE synthesis reactors to eliminate the need for catalytic distillation columns or reactive distillation columns. However, the isothermal multi-tubular reactors require high capital expenditure, thus, not resolving the issues related to the high production costs for MTBE.

Overall, while systems and methods for producing MTBE exist, the need for improvements in this field persists in light of at least the aforementioned drawbacks for the conventional systems and methods.

### BRIEF SUMMARY OF THE INVENTION

A solution to at least the above mentioned problems associated with the systems and methods for producing MTBE from isobutylene and methanol has been discovered. The solution resides in a method for producing MTBE using a system that includes at least three MTBE synthesis reactors. Notably, the first and the second MTBE synthesis reactors are operated in parallel with the third MTBE synthesis reactor operated in series with the first and second MTBE synthesis reactors. This can be beneficial for at least increasing the MTBE concentration overall, specifically the MTBE concentration in the product effluent stream from the third MTBE synthesis reactor. Furthermore, the disclosed system does not include a super fractionator, a catalytic distillation column (reactive distillation column), or isothermal reactors, resulting in reduced capital expenditure and/or operating cost for producing MTBE, compared to conventional MTBE production systems. Overall, in the disclosed methods, an optimum volume of methanol stream required to maximize MTBE production and reduce slippage of isobutylene to minimum acceptable values together with a crude C₄ stream are flowed into a primary reaction unit that comprises a first reactor and a second reactor in parallel configured to produce maximum values of final MTBE volumes under higher or equal established purity commercial quality specifications levels. Therefore, the system and method of the present invention provide a technical solution to at least some of the problems associated with the conventional systems and methods for producing MTBE, as mentioned above.

The method according to the invention is defined in the claims. Embodiments of the invention include a method of producing methyl tertiary butyl ether. The method comprises feeding isobutylene and methanol to a first reactor and a second reactor, arranged in parallel. The method comprises subjecting the isobutylene and the methanol, in the first reactor and the second reactor, respectively, to reaction conditions sufficient to cause the isobutylene to react with the methanol to produce a first portion of MTBE in effluent from the first reactor and in effluent from the second reactor. The method comprises combining the effluent from the first reactor and the effluent from the second reactor to form a combined reactor effluent stream. The combined reactor effluent stream further comprises isobutylene. The method comprises reacting the isobutylene comprised in a first portion of the combined reactor effluent stream with methanol in a third reactor that is in series with the first reactor and second reactor, to produce a third reactor effluent stream comprising a second portion of MTBE. The method comprises mixing a second portion of the combined reactor effluent stream with the third reactor effluent stream to form a mixed intermediate product stream. The method comprises recycling a third portion of the combined effluent stream to the first reactor and the second reactor. The method further comprises separating the mixed intermediate product stream to form a product stream comprising primarily MTBE, a stream comprising primarily methanol, and a C₄ raffinate stream. In the method according to the invention , the step of feeding isobutylene and methanol to the first reactor and the second reactor comprises: mixing a crude C4 stream comprising isobutylene with methanol to form a feed stream; splitting the feed stream into a first feed stream and a second feed stream; and
feeding the first feed stream to the first reactor and feeding the second feed stream to the second reactor. The method according to the invention does not include a separation step that utilizes super fractionator column or catalytic distillation column.

Embodiments of the invention include a method of producing methyl tertiary butyl ether. The method includes mixing a crude C₄ stream comprising isobutylene with methanol to form a feed stream. The method includes splitting the feed stream to form a first feed stream and a second feed stream. The method includes feeding the first feed stream to a first adiabatic fixed bed reactor and feeding the second feed stream to a second adiabatic fixed bed reactor. The method includes subjecting the isobutylene and the methanol, in the first adiabatic fixed bed reactor and the second adiabatic fixed bed reactor, respectively, to reaction conditions sufficient to cause the isobutylene to react with the methanol to produce a first portion of MTBE in effluent from the first adiabatic fixed bed reactor and in effluent from the second adiabatic fixed bed reactor. The method includes combining the effluent from the first adiabatic fixed bed reactor and the effluent from the second adiabatic fixed bed reactor to form a combined reactor effluent stream. The combined reactor effluent stream further comprises isobutylene. The method includes reacting the isobutylene comprised in a first portion of the combined reactor effluent stream with methanol in a third adiabatic fixed bed reactor that is in series with the first adiabatic fixed bed reactor and second adiabatic fixed bed reactor, to produce a third adiabatic fixed bed reactor effluent stream comprising a second portion of MTBE. The method includes mixing a second portion of the combined reactor effluent stream with the third adiabatic fixed bed reactor effluent stream to form a mixed intermediate product stream. The method includes recycling a third portion of the combined effluent stream to the first adiabatic fixed bed reactor and the second adiabatic fixed bed reactor. The method includes separating the mixed intermediate product stream to form a stream comprising primarily MTBE, a stream comprising primarily methanol, and a C₄ raffinate stream.

Embodiments of the invention include a method of producing methyl tertiary butyl ether. The method includes mixing a crude C₄ stream comprising isobutylene with methanol to form a feed stream. The method includes splitting the feed stream to form a first feed stream and a second feed stream. The method includes feeding the first feed stream to a first adiabatic fixed bed reactor and feeding the second feed stream to a second adiabatic fixed bed reactor. The method includes subjecting the isobutylene and the methanol, in the first adiabatic fixed bed reactor and the second adiabatic fixed bed reactor, respectively, to reaction conditions sufficient to cause the isobutylene to react with the methanol to produce a first portion of MTBE in effluent from the first adiabatic fixed bed reactor and in effluent from the second adiabatic fixed bed reactor. The method includes combining effluent from the first adiabatic fixed bed reactor and effluent from the second adiabatic fixed bed reactor to form a stream comprising MTBE, water, isobutylene. The method includes separating water from the stream comprising MTBE, water, isobutylene to form a combined reactor effluent stream. The method includes reacting the isobutylene comprised in a first portion of the combined reactor effluent stream with methanol in a third adiabatic fixed bed reactor that is in series with the first adiabatic fixed bed reactor and second adiabatic fixed bed reactor, to produce a third adiabatic fixed bed reactor effluent stream comprising a second portion of MTBE. The method includes mixing a second portion of the combined reactor effluent stream with the third adiabatic fixed bed reactor effluent stream to form a mixed intermediate product stream. The method includes recycling a third portion of the combined effluent stream to the first adiabatic fixed bed reactor and the second adiabatic fixed bed reactor. The method further includes separating the mixed intermediate product stream to form a stream comprising primarily MTBE, a stream comprising primarily methanol, and a C₄ raffinate stream.

The following includes definitions of various terms and phrases used throughout this specification.

The terms "about" or "approximately" are defined as being close to as understood by one of ordinary skill in the art. In one non-limiting embodiment the terms are defined to be within 10%, preferably, within 5%, more preferably, within 1%, and most preferably, within 0.5%.

The terms "wt.%", "vol.%" or "mol.%" refer to a weight, volume, or molar percentage of a component, respectively, based on the total weight, the total volume, or the total moles of material that includes the component. In a non-limiting example, 10 moles of component in 100 moles of the material is 10 mol.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification, include any measurable decrease or complete inhibition to achieve a desired result.

The term "effective," as that term is used in the specification and/or claims, means adequate to accomplish a desired, expected, or intended result.

The use of the words "a" or "an" when used in conjunction with the term "comprising," "including," "containing," or "having" in the claims or the specification may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The process of the present invention can "comprise," "consist essentially of," or "consist of" particular ingredients, components, compositions, etc., disclosed throughout the specification.

The term "primarily," as that term is used in the specification and/or claims, means greater than any of 50 wt.%, 50 mol.%, and 50 vol.%. For example, "primarily" may include 50.1 wt.% to 100 wt.% and all values and ranges there between, 50.1 mol.% to 100 mol.% and all values and ranges there between, or 50.1 vol.% to 100 vol.% and all values and ranges there between.

Features from one embodiment may be combined with features from any of the other embodiments. In further embodiments, additional features may be added to the specific embodiments described herein.

### BRIEF DESCRIPTION OF THE DRAWINGS

For a more complete understanding, reference is now made to the following descriptions taken in conjunction with the accompanying drawings, in which:
FIG. 1 shows a schematic diagram of a system for producing MTBE, according to embodiments of the invention;
FIG. 2 shows a schematic flowchart of a method of producing MTBE, according to embodiments of the invention;
FIG. 3A shows correlation between isobutylene conversion rate of an MTBE production process and time on stream when the reactor is operated with and without recycling part of MTBE product back to the reactor;
FIG. 3B shows correlation between MTBE selectivity of an MTBE production process and time on stream when the reactor is operated with and without recycling part of MTBE product back to the reactor; and
FIG. 4 shows correlation between isobutylene conversion rate of an MTBE production process and time on stream with different MTBE concentrations in the feed stream into the MTBE synthesis reactor.

### DETAILED DESCRIPTION OF THE INVENTION

Currently, MTBE can be produced by reacting isobutylene with methanol in an MTBE reactor followed by further MTBE synthesis reaction in a catalytic distillation column or a reactive distillation column. Other MTBE systems use super fractionator for separation of light ends (C₄ and methanol) from MTBE. Therefore, the capital expenditure and the operating costs for producing MTBE are relatively high, resulting in high production costs for MTBE. The present invention provides a solution to the problem. The solution is premised on a method for producing MTBE that includes reacting isobutylene and methanol in a first reactor and second reactor, which are operated in parallel. At least a portion of the combined effluent stream from the first reactor and second reactor is further subjected to reaction conditions for producing additional MTBE in a third reactor that is in series with the first reactor and the second reactor. The disclosed method and system do not require the operation of a catalytic distillation column, a reactive distillation column, a super fractionator, or an isothermal reactor, resulting in reduced capital expenditure and/or operating costs compared to conventional MTBE production systems. These and other non-limiting aspects of the present invention are discussed in further detail in the following sections.

### A. System for producing MTBE

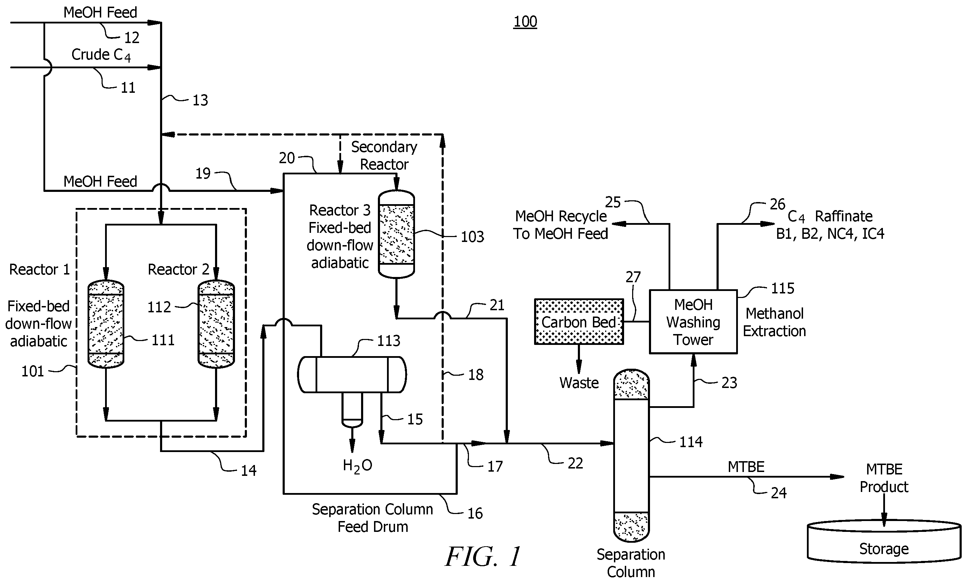

In embodiments of the system for producing MTBE according to the method of the invention, the system includes a primary reaction unit operated with a secondary reaction unit. The primary reaction unit can include two reactors operated in parallel. The second reaction unit may include a third reactor in series with the primary reaction unit. With reference to FIG. 1, a schematic diagram is shown for system 100, which is configured for producing MTBE using isobutylene and methanol.

According to embodiments of the system, system 100 comprises primary reaction unit 101 configured to react methanol and isobutylene to produce MTBE. In embodiments of the system, primary reaction unit 101 includes first reactor 111 and second reactor 112. First reactor 111 and second reactor 112 may be operated in parallel. In embodiments of the system, isobutylene that is flowed into primary reaction unit 101 is from crude C₄ stream 11. Methanol that is flowed into primary reaction unit 101 may be from crude methanol stream 12. Crude C₄ stream 11 may comprise isobutylene, 1-butene, 2-butene, n-butane, isobutane, 1,3-butadiene, or combinations thereof. In system 100, crude C₄ stream 11 and first methanol stream 12 may be combined to form combined feed stream 13. Combined feed stream 13 can be flowed into first reactor 111 and second reactor 112. In embodiments of the system, system 100 can optionally include a feed preheater configured to heat combined feed stream 13 to a predetermined feed temperature. The predetermined feed temperature may be in a range of 37 to 47 °C, preferably at 42 °C.

First reactor 111 and second reactor 112 can each individually include an adiabatic fixed bed reactor. In embodiments of the system, first reactor 111 and second reactor 112 each individually include a down-flow reactor. First reactor 111 and second reactor 112 may each include a catalyst. The catalyst may be a strongly acidic resin comprising polystyrene based resin, polystyrene divinyl benzene based resin, sulfonic resin, macroreticular resin, acidic ion-exchange resin, sulphonated macroporous resin, or any combination thereof. In embodiments of the system, first reactor 111 and/or second reactor 112 may include a catalyst support grid (e.g., a Johnson screen) configured to provide support to the catalyst and minimize catalyst carry-over in the effluents. In embodiments of the system, effluent from first reactor 111 and effluent from second reactor 112 are combined to form combined stream 14. Combined stream 14 may comprise MTBE, and unreacted isobutylene. Combined stream 14 may further include water, methanol, 1-butene, methyl-secondary butyl ether (MSBE), 2,4,4-Trimethyl-1-pentene (244TM1P), 2,4,4-Trimethyl-2-pentene (244TM2P), isobutane, n-butane, 1,3-butadiene, cis-2-butene, trans-2-butene, 1,3-Cyclopentadiene (CD13), 1,,3-Pentadiene (14PD), or any combinations thereof. In embodiments of the system, system 100 may include an after-cooler configured to cool the effluent from first reactor 111 and/or the effluent from second reactor 112 before forming combined stream 14.

According to embodiments of the system, an outlet of primary reaction unit 101, including outlets of first reactor 111 and second reactor 112, is in fluid communication with separation column feed drum 113 such that combined stream 14 flows from primary reaction unit 101 to separation column feed drum 113. In embodiments of the system, separation column feed drum 113 is configured to remove at least some water from combined stream 14 to form combined reactor effluent stream 15 comprising MTBE and unreacted isobutylene. Separation column feed drum 113 may be further configured to control pressure and/or vapor content in primary reaction unit 101. Separation column feed drum 113, in embodiments of the system, may include a nitrogen blanket configured to control pressure therein. Combined reactor effluent stream 15, in embodiments of the system, can be divided to form first portion 16, second portion 17, and/or third portion 18.

In embodiments of the system, an outlet of separation column feed drum 113 may be in fluid communication with an inlet of third reactor 103 such that first portion 16 of combined reactor effluent stream 15 flows from separation column feed drum 113 to third reactor 103. First portion 16 of combined reactor effluent stream 15 may be combined with second methanol stream 19 to form second feed stream 20 for third reactor 103. In embodiments of the system, third reactor 103 is configured to carry out reaction between unreacted isobutylene and methanol of second feed stream 20 for producing MTBE in third reactor effluent stream 21. Third reactor 103 may include an adiabatic fixed bed reactor. The adiabatic fixed bed reactor can be a down flow reactor. In embodiments of the system, third reactor 103 may include a strong-acidic catalyst comprising polystyrene based resin, polystyrene divinyl benzene based resin, sulfonic resin, macroreticular resin, acidic ion-exchange resin, sulphonated macroporous resin, or any combination thereof. The strong acidic catalyst can include a sulfonated polystyrene cross-linked resin. The sulfonated polystyrene cross-linked resin catalyst can be a Amberlyst^{™} catalyst from DUPONT (USA) including Amberlyst^{™} 15 (A-15), Amberlyst^{™} 35 (A-35), Amberlyst^{™} 36 (A-36), Amberlyst^{™} 40 (A-40), Amberlyst ^{™} 48 (A-48), or combinations thereof. Additional examples of the sulfonated polystyrene cross-linked resin can include CT-175, CT-252, CT-275, and combinations thereof (Purolite^{®}, USA).

In embodiments of the system, an outlet of separation column feed drum 113 may be in fluid communication with an inlet of primary reaction unit 101 such that third portion 18 of combined reactor effluent stream 15 flows from separation column feed drum 113 to primary reaction unit 101. Third portion 18 of combined reactor effluent stream 15 may be combined with combined feed stream 13 before being flowed into primary reaction unit 101.

According to embodiments of the system, second portion 17 of combined reactor effluent stream 15 and third reactor effluent stream 21 can be combined to form mixed intermediate product stream 22. In embodiments of the system, system 100 includes separation column 114 configured to separate mixed intermediate product stream 22 to form (i) top stream 23 including methanol, C₄ hydrocarbons, and waste, and (ii) product stream 24 comprising primarily MTBE. In embodiments of the system, separation column 114 can include a distillation column. Separation column 114 may not include super fractionator. In embodiments of the system, separation column 114 may include a feed filter configured to filter mixed intermediate product stream 22 before it is flowed into separation column 114. Separation column 114 may further include a heat exchanger configured to heat mixed intermediate product stream 22 before it is flowed into separation column 114 using product stream 24 as a heating medium. In embodiments of the system, separation column 114 is configured to utilize medium pressure steam as a heating medium for a reboiler thereof. In embodiments of the system, the reboiler of separation column 114 may include a vertical thermosiphon exchanger, and a reboiler de-superheater configured to de-superheat the medium pressure steam before it enters the reboiler.

According to embodiments of thesystem, an outlet of separation column 114 is in fluid communication with methanol washing tower 115 such that top stream 23 flows from separation column 114 to methanol washing tower 115. Methanol washing tower 115, in embodiments of the system, is configured to separate top stream 23 to produce recycle methanol stream 25 comprising primarily methanol, and C₄ raffinate stream 26 comprising primarily C₄ hydrocarbons, and waste stream 27. Waste stream 27 may be further processed in carbon bed to remove total organic carbon (TOC) before water discharge. In embodiments of the system, methanol washing tower 115 may include a raffinate wash column and a methanol enrichment column. The raffinate wash column can be configured to extract methanol from top stream 23 using a countercurrent stream of water. The methanol enrichment column is configured to separate wash water from the methanol of a bottom stream of the raffinate wash column. The methanol enrichment column may comprise trays and downcomers. In embodiments of the system, a bottom stream of the methanol enrichment column can be pumped back to the raffinate wash column with some fresh condensate to make up for water loss. An overhead stream of the methanol enrichment column can be recycled as part of methanol feed for system 100.

### B. Method of producing MTBE

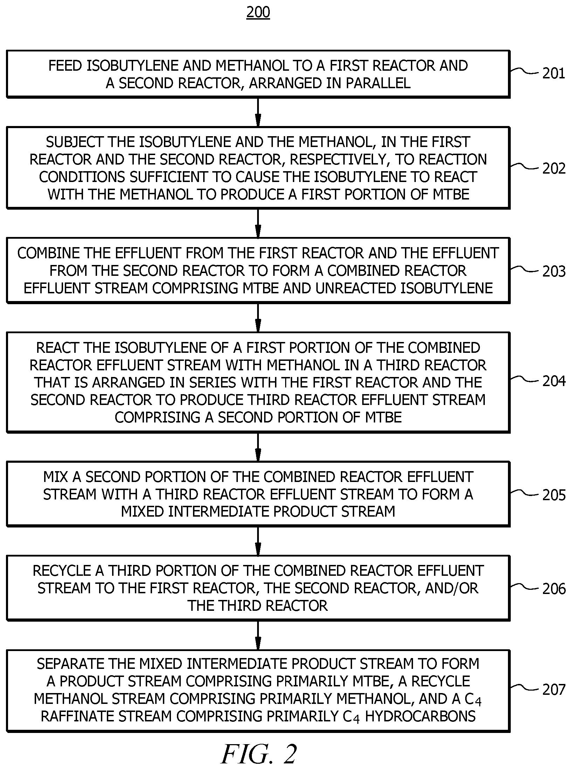

Methods for producing MTBE from isobutylene and methanol with a reduced production cost compared to conventional methods have been discovered. As shown in FIG. 2, embodiments of the invention include method 200 for producing methyl tertiary (tert) butyl ether (MTBE). Method 200 may be implemented by system 100, as shown in FIG. 1, and described above.

According to embodiments of the invention, as shown in block 201, method 200 incudes feeding isobutylene and methanol to first reactor 111 and second reactor 112, arranged in parallel. In embodiments of the invention, at block 201, isobutylene is supplied from crude C₄ stream 11 comprising isobutylene, 1-butene, 2-butene, n-butane, isobutane, 1,3-butadiene, or combinations thereof. Crude C₄ stream 11 may be from a C₄ raffinate of a steam cracking unit or from a fluid catalytic cracking unit. In embodiments of the invention, crude C₄ stream may include 10 to 40 wt.% isobutylene. First methanol stream may comprise 98 to 99.9 wt.% methanol.

In embodiments of the invention, feeding at block 201 can include mixing crude C₄ stream 11 with first methanol stream 12 to form combined feed stream 13. Mixing at block 201 may be carried out at a molar ratio of first methanol stream to isobutylene in crude C₄ stream to first methanol stream in a range of 1 to 1.3 and all ranges and values there between including ranges of 1 to 1.1, 1.1 to 1.2, and 1.2 to 1.3. Combined feed stream 13 may contain an excessive amount of methanol adapted to overcome an azeotropic limit in an overhead of separation column 114. Feeding at block 201 can further include optionally heating combined feed stream 13 to a predetermined temperature. The predetermined temperature may be in a range of 35 to 47 °C, preferably at about 42 °C and all ranges and values there between including ranges of 35 to 37 °C, 37 to 39 °C, 39 to 41 °C, 41 to 43 °C, 43 to 45 °C, and 45 to 47 °C. In embodiments of the invention, feeding at block 201 may further include splitting combined feed stream 13 to produce a first feed stream and a second feed stream. Feeding at block 201, according to embodiments of the invention, may further still include feeding the first feed stream to first reactor 111 and feeding the second feed stream to second reactor 112.

According to embodiments of the invention, as shown in block 202, method 200 includes subjecting the isobutylene and the methanol, in first reactor 111 and second reactor 112, respectively, to reaction conditions sufficient to cause the isobutylene to react with the methanol to produce a first portion of MTBE in effluent from first reactor 111 and in effluent from second reactor 112. In embodiments of the invention, reaction conditions in first reactor 111 and/or second reactor 112 include a reaction temperature of 40 to 60 °C and all ranges and values there between including ranges of 40 to 42 °C, 42 to 44 °C, 44 to 46 °C, 46 to 48 °C, 48 to 50 °C, 50 to 52 °C, 52 to 54 °C, 54 to 56 °C, 56 to 58 °C, and 58 to 60 °C. Reaction conditions in first reactor 111 and/or second reactor 112 may include an operating pressure of 6 to 10 bar and all ranges and values there between including ranges of 6 to 6.5 bar, 6.5 to 7.0 bar, 7.0 to 7.5 bar, 7.5 to 8.0 bar, 8.0 to 8.5 bar, 8.5 to 9.0 bar, 9.0 to 9.5 bar, and 9.5 to 10 bar. In embodiments of the invention, reaction conditions in first reactor 111 and/or second reactor 112 include a weight hourly space velocity in a range of 3 to 8 hr⁻¹ and all ranges and values there between including ranges of 3 to 4 hr⁻¹, 4 to 5 hr⁻¹, 5 to 6 hr⁻¹, 6 to 7 hr⁻¹, and 7 to 8 hr⁻¹. According to embodiments of the invention, the effluent from first reactor 111 and/or the effluent from second reactor 112 comprises 16 to 56 wt.% MTBE, and 0 to 4 wt.% unreacted isobutylene. The effluent from first reactor 111 and/or the effluent from second reactor 112 may comprise 60 to 90 wt.% C₄ hydrocarbons other than isobutylene. In embodiments of the invention, first reactor 111 and/or second reactor 112 are operated such that an isobutylene conversion rate of 94% is achieved by primary reaction unit 101.

According to embodiments of the invention, as shown in block 203, method 200 includes combining the effluent from first reactor 111 and the effluent from second reactor 112 to form combined reactor effluent stream 15 comprising MTBE and unreacted isobutylene. In embodiments of the invention, the effluent from first reactor 111 and/or the effluent from second reactor 112 comprises water, and the combining step at block 203 comprises combining the effluent stream from first reactor 111 and the effluent from second reactor 112 to form combined stream 14 comprising MTBE, water, and isobutylene, and separating water in separation column feed drum 113 from combined stream 14 comprising MTBE, water, and isobutylene to form combined reactor effluent stream 15. The separation column feed drum can be operated at a temperature of 50 to 55 °C and a pressure of 4 to 6 bar.

According to embodiments of the invention, as shown in block 204, method 200 includes reacting the isobutylene of first portion 16 of combined reactor effluent stream 15 with methanol of second methanol stream 19 in third reactor 103 that is in series with first reactor 111 and second reactor 112, to produce third reactor effluent stream 21 comprising a second portion of MTBE. At block 204, third reactor 103 can be operated at an operating temperature of 40 to 65 °C and all ranges and values there between including ranges of 40 to 45 °C, 45 to 50 °C, 50 to 55 °C, 55 to 60 °C, and 60 to 65 °C. At block 204, third reactor 103 can be operated at an operating pressure of 6 to 10 bar and all ranges and values there between including ranges of 6 to 7 bar, 7 to 8 bar, 8 to 9 bar, and 9 to 10 bar. At block 204, third reactor 103 can be operated with a weight hourly space velocity in a ranges of 3 to 8 hr⁻¹ and all ranges and values there between including ranges of 3 to 4 hr⁻¹, 4 to 5 hr⁻¹, 5 to 6 hr⁻¹, 6 to 7 hr⁻¹, and 7 to 8 hr⁻¹. In embodiments of the invention, third reactor effluent stream 21 comprises 16 to 56 wt.% MTBE and all ranges and values there between including ranges of 16 to 20 wt.%, 20 to 24 wt.%, 24 to 28 wt.%, 28 to 32 wt.%, 32 to 36 wt.%, 36 to 40 wt.%, 40 to 44 wt.%, 44 to 48 wt.%, 48 to 52 wt.%, and 52 to 56 wt.%.. In embodiments of the invention, third reactor 103 is operated to convert about 6% more isobutylene from first portion 16 of combined reactor effluent stream 15. In embodiments of the invention, an overall conversion rate of 97 to 100% for isobutylene can be achieved at block 204. In embodiments of the invention, first reactor 111, second reactor 112, and third reactor 103 can be operated under substantially the same, or different operating conditions, which include operating temperature, operating pressure, weight hourly space velocity, or any combinations thereof. In embodiments of the invention, third portion 18 of combined reactor effluent stream 15 may be cooled to a predetermined temperature before it is flowed into third reactor 103. The predetermined temperature for third portion 18 of combined reactor effluent stream 15 may be about 45 °C. In embodiments of the invention, at least some of fresh crude C₄ stream including isobutylene is added into first portion 16 of combined reactor effluent stream 15 before it is flowed into third reactor 103.

According to embodiments of the invention, as shown in block 205, method 200 includes mixing second portion 17 of combined reactor effluent stream 15 with third reactor effluent stream 21 to form mixed intermediate product stream 22. According to embodiments of the invention, as shown in block 206, method 200 includes recycling third portion 18 of combined reactor effluent stream 15 to first reactor 111, second reactor 112, and/or third reactor 103. A volumetric ratio between third portion 18 of combined reactor effluent stream 15 to combined feed stream 13 may be in a range of 2 to 4 (i.e., the recycle/fresh feed ratio is 2-4.). At block 206, third portion 18 of combined effluent stream 15 may be combined with combined feed stream 13 before being flowed into first reactor 111 and/or second reactor 112. A volumetric ratio of third portion 18 that is flowed into first reactor 111 and second reactor 112 to combined feed stream 13 may be in a range of about 2.5.

According to embodiments of the invention, as shown in block 207, method 200 includes separating mixed intermediate product stream 22 to form product stream 24 comprising primarily MTBE, recycle methanol stream 25 comprising primarily methanol, and C₄ raffinate stream 26 comprising primarily C₄ hydrocarbons. Product stream 24 may comprise at least 98 wt.% MTBE. In embodiments of the invention, separating at block 207 can include separating mixed intermediate product stream 22 in separation column 114 to produce top stream 23 comprising methanol and C₄ hydrocarbons, and product stream 24 comprising primarily MTBE. Separation column 114 may be operated at an overhead temperature range of 45 to 60 °C and a bottom (or reboiler) temperature range of 135 to 150 °C. Separation column 114 may be operated at an operating pressure of 7 to 8 bar. Separating at block 207 may further include processing top stream 23 in methanol washing tower 115 to produce recycle methanol stream 25, C₄ raffinate stream 26, and/or waste stream 27 comprising waste water with TOC (total organic carbon). The processing step in methanol washing tower 115 may further produce residual methanol, which is recycled to primary reaction unit 101 and/or third reactor 103. In embodiments of the invention, the raffinate wash column of methanol washing tower 115 can be operated at a temperature of 40 to 45 °C, a pressure of 13 to 15 bar. The methanol enrichment column of methanol washing tower 115 can be operated at a temperature of 80 to 135 °C and an operating pressure of 2 to 3 bar. In embodiments of the invention, product stream 24 can be cooled in the heat exchanger of separation column 114 and/or in a product cooler to achieve a battery limit temperature.

According to embodiments of the invention, including MTBE in a feed stream into an MTBE synthesis reaction unit (e.g., first reactor 111, second reactor 112, and/or third reactor 113) improves conversion rate of isobutylene for MTBE production compared to no MTBE in the feed stream into the MTBE synthesis reaction unit. In embodiments of the invention, the isobutylene conversion rate increases with an increasing MTBE concentration in the feed stream into the MTBE synthesis reaction unit. The improvement of isobutylene conversion rate may be due to impact of MTBE on catalyst activity, which is caused by structural change of the catalyst, and optimization of exothermic nature of the MTBE synthesis reaction. In embodiments of the invention, the isobutylene conversion rate and the MTBE concentration in the feed stream to an MTBE synthesis reaction unit can have a substantially linear correlation.

Although embodiments of the present invention have been described with reference to blocks of FIG. 2 should be appreciated that operation of the present invention is not limited to the particular blocks and/or the particular order of the blocks illustrated in FIG. 2. Accordingly, embodiments of the invention may provide functionality as described herein using various blocks in a sequence different than that of FIG. 2.

The systems and processes described herein can also include various equipment that is not shown and is known to one of skill in the art of chemical processing. For example, some controllers, piping, computers, valves, pumps, heaters, thermocouples, pressure indicators, mixers, heat exchangers, and the like may not be shown.

As part of the disclosure of the present invention, specific examples are included below. The examples are for illustrative purposes only and are not intended to limit the invention. The present examples are outside of the scope of present claim 1 and are for reference only.

### EXAMPLE 1

### (Mass Balance for an MTBE production system)

Simulations were conducted in Pro II platform for MTBE production in a system disclosed above. A first methanol stream at about 46 °C and a C₄ raffinate stream at 40 °C were fed into the system, as described above. The system did not include a catalytic distillation column, a reactive distillation column, or a super fractionator. Results for the composition of process streams and product stream are shown in Table 1.

**Table 1. Mass Balance for MTBE production system**

| ***Components (Mole %)*** | **Methanol Feed** | **C4 Raffinate Feed** | **Feed to Separation Column** | **Separation Column Overhead** | **Separation Column Bottom** |
|---|---|---|---|---|---|
| MTBE | | | 36.822 | 8.774 | 98.556 |
| ISOBUTENE | | 43.72 | 2.324 | 3.38 | 0 |
| METHANOL | 100 | | 9.272 | 13.484 | 0 |
| 1-BUTENE | | 30.614 | 27.757 | 40.368 | 0 |
| MSBE | | | 0.12 | 0.003 | 0.378 |
| 244TM1P | | | 0.166 | 0 | 0.533 |
| 244TM2P | | | 0.166 | 0 | 0.533 |
| ISOBUTANE | | 3.802 | 3.462 | 5.035 | 0 |
| N-BUTANE | | 4.602 | 4.191 | 6.095 | 0 |
| 1,3-BUTADIENE | | 0.3 | 0.273 | 0.397 | 0 |
| Cis-2-BUTENE | | 7.003 | 6.377 | 9.274 | 0 |
| Trans-2-BUTENE | | 9.704 | 8.837 | 12.852 | 0 |
| CD13 | | 0.15 | 0.137 | 0.199 | 0 |
| 14PD | | 0.105 | 0.096 | 0.139 | 0 |
| WATER | | | | | |

| **Total Flow** | | | | | |
|---|---|---|---|---|---|
| Kg/hr | 28.966 | 100 | 128.966 | 75.095 | 53.871 |
| Temperature (°C) | 46 | 40 | 40 | 67.996 | 135.348 |
| Pressure (Kg/cm²) | 8 | 8 | 8 | 8 | 8 |

The results show that the system was capable of producing an MTBE product stream comprising more than 98 wt.% MTBE without using a catalytic (reactive) distillation column and a super fractionator.

### EXAMPLE 2

### (Effects of MTBE Recycle on Isobutylene Conversion Rate)

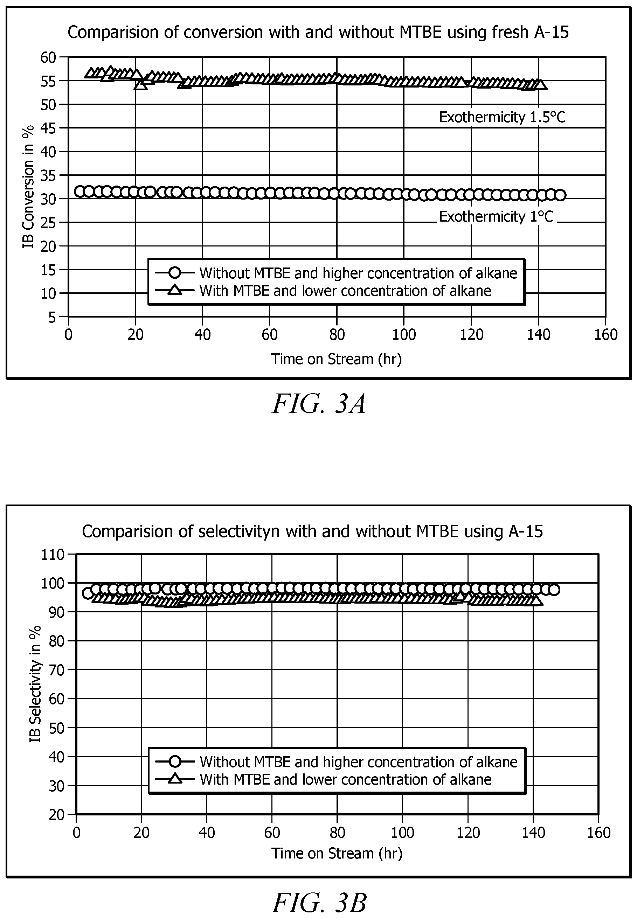

Experiments were conducted to investigate effects of MTBE recycle stream on the conversion rate of isobutylene and selectivity of MTBE in an MTBE synthesis reactor. The reaction conditions used in the experiments included a reaction temperature of 60 °C, and a weight hourly space velocity of 72.49 hr⁻¹. The C₄ raffinate feed was flowed into the reactor at a flow rate of 53.8 ml/hr. Methanol was flowed into the reactor at a flow rate of 0.1 to achieve an isobutene to methanol molar ratio of about 1:1. The catalyst used in the reactor was about 0.50 g. The correlation between isobutylene conversion rate (%) and time on stream (reaction time, hr) was plotted in FIG. 3A for reactors operated with and without MTBE recycle. The correlation between MTBE selectivity (%) and time on stream (reaction time, hr) was plotted in FIG. 3B for reactors operated with and without MTBE recycle.

The results indicate that MTBE production processes that include recycling part of MTBE product stream back to the reactor show about 83% improvement in isobutylene conversion rate compared to the conversion rate achieved by MTBE production processes that does not use MTBE recycle stream. This is due to impact of MTBE on catalyst activity, which is caused by structural change of the catalyst, and optimization of exothermic nature of the MTBE synthesis reaction. However, the selectivity of MTBE does not show any difference between MTBE production processes that recycled part of MTBE product stream back to the reactor and MTBE production processes that does not use MTBE recycle stream.

### EXAMPLE 3

### (Effects of MTBE Concentrations in MTBE Feed Stream on Isobutylene Conversion rate)

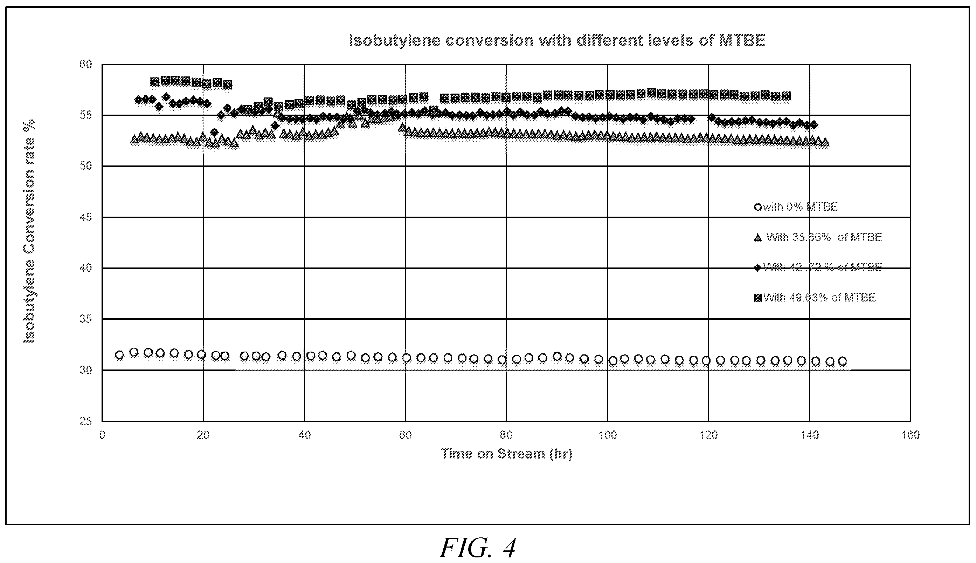

Simulations were conducted using PRO II platform to obtain compositions of a feed stream flowed into a reactor for MTBE synthesis. The compositions of the feed stream flowed into reactor for MTBE synthesis are shown in Table 2. Experiments were then conducted using the feed stream compositions obtained via the simulations. For the experiments, the catalyst (A-15) quantity used was about 0.5 g. The reaction temperature for MTBE synthesis was 60 °C and the weight hourly space velocity used for MTBE synthesis experiments was about 73 hr⁻¹. The methanol to isobutylene molar ratio fed into the system was 1:1. The results for the MTBE conversion rate against time on stream (hr) for each feed stream composition are shown in FIG. 4A and Table 3.

**Table 2. Feed stream compositions flowed into the primary reaction unit for MTBE synthesis**

| Component s | Wt. % for Composition 1 | Wt. % for Composition 2 | Wt. % for Composition 3 | Wt. % for Composition 4 |
|---|---|---|---|---|
| n-butane | 2.61 | 0.94 | 0.31 | 0.89 |
| isobutane | 54.29 | 37.31 | 19.74 | 28.77 |
| trans-2-butene | 2.71 | 0.17 | 0.26 | 0.13 |
| Methanol | 13.6 | 7.94 | 12.58 | 6.54 |
| isobutylene | 21.44 | 14.6 | 24.23 | 11.28 |
| Cis -2 - butene | 2.70 | 0.14 | 0.24 | 0.11 |
| 1,3-butadiene | 2.65 | 0.5 | 0.22 | 0.39 |
| **MTBE** | **0** | **35.66** | **42.72** | **49.63** |
| 1-butene | 0 | 2.32 | 0 | 1.80 |
| propane | 0 | 0.34 | 0 | 0.30 |
| propylene | 0 | 0.12 | 0 | 0.16 |

**Table 3. Isobutene (isobutylene) conversion rate for each MTBE concentration in feed stream**

| Time (Hrs) | MTBE concentration in feed (Wt. %) | | | |
|---|---|---|---|---|
| | 0.00 | 35.66 | 42.72 | 49.63 |
| | Isobutylene Conversion Rate (Wt. %) | | | |
| 20 | 31.54 | 52.92 | 56.39 | 58.10 |
| 60 | 31.21 | 53.45 | 55.19 | 56.60 |
| 100 | 30.93 | 53.11 | 54.91 | 57.06 |
| 140 | 30.88 | 52.64 | 54.03 | 56.92 |
| Average | 31.14 | 53.03 | 55.13 | 57.17 |

The results from FIG. 4 indicate that the isobutylene conversion rate increases with increasing MTBE concentration in feed stream. The results from Table 3 were also analyzed via linear regression. The results indicate that the conversion rate for isobutylene (y) can be described by MTBE concentration (x; wt.%) in feed stream as y=0.5441x+31.706 (R²=0.9854). This is due to impact of MTBE on catalyst activity, which is caused by structural change of the catalyst, and optimization of exothermic nature of the MTBE synthesis reaction.

In the context of the present invention, at least the following embodiments are described. Embodiment 1 is a method of producing methyl tertiary butyl ether (MTBE). The method includes feeding isobutylene and methanol to a first reactor and a second reactor, arranged in parallel and subjecting the isobutylene and the methanol, in the first reactor and the second reactor, respectively, to reaction conditions sufficient to cause the isobutylene to react with the methanol to produce a first portion of MTBE in effluent from the first reactor and in effluent from the second reactor. The method further includes combining effluent from the first reactor and effluent from the second reactor to form a combined reactor effluent stream, wherein the combined reactor effluent stream further contains isobutylene. The method still further includes reacting the isobutylene contained in a first portion of the combined reactor effluent stream with methanol in a third reactor that is in series with the first reactor and second reactor, to produce a third reactor effluent stream containing a second portion of MTBE. The method also includes mixing a second portion of the combined reactor effluent stream with the third reactor effluent stream to form a mixed intermediate product stream and recycling a third portion of the combined effluent stream to the first reactor and the second reactor. In addition, the method includes separating the mixed intermediate product stream to form a product stream containing primarily MTBE, a stream containing primarily methanol, and a C₄ raffinate stream. Embodiment 1 also comprises the step of feeding isobutylene and methanol to the first reactor and the second reactor includes mixing a crude C₄ stream containing isobutylene with methanol to form a feed stream and splitting the feed stream into a first feed stream and a second feed stream. The method further includes feeding the first feed stream to the first reactor and feeding the second feed stream to the second reactor. In embodiment 1 , the method does not include a separation step that utilizes a super fractionator column or catalytic distillation column. Embodiment 2 is the method of embodiment 1, wherein the first reactor, the second reactor, and/or the third reactor each individually include an adiabatic fixed bed reactor. Embodiment 3 is the method of either of embodiments 1 or 2, wherein the first reactor includes an adiabatic fixed bed reactor. Embodiment 4 is the method of any of embodiments 1 to 3, wherein the first reactor effluent stream and the second reactor effluent stream further contains includes combining the effluent from the first reactor and the effluent from the second reactor to form a stream containing MTBE, water, isobutylene. The method further includes separating water from the stream containing MTBE, water, isobutylene to form the combined reactor effluent stream. Embodiment 5 is the method of any of embodiments 1 to 4, wherein the first reactor and/or the second reactor each individually include a down flow reactor. Embodiment 6 is the method of any of embodiments 1 to 5, wherein the first reactor and the second reactor each comprise a polystyrene based resin. Embodiment 7 is the method of any of embodiments 1 to 6, wherein the product stream contains at least 98 wt.% MTBE. Embodiment 8 is the method of any of embodiments 1 to 7, wherein the third reactor is operated at a higher pressure than the first reactor and the second reactor. Embodiment 9 is the method of any of embodiments 1 to 5 and 7 to 8, wherein the first reactor and the second reactor each include a catalyst that contains polystyrene based resin, polystyrene divinyl benzene based resin, sulfonic resin, macroreticular resin, acidic ion-exchange resin, sulphonated macroporous resin, or any combination thereof. Embodiment 10 is the method of any of embodiments 1 to 9, wherein the first reactor and the second reactor are each operated at an operating temperature in a range 40 to 60 °C. Embodiment 11 is the method of any of embodiments 1 to 10, wherein the first reactor and the second reactor are each operated at an operating pressure in a range of 6 to 10 bar. Embodiment 12 is the method of any of embodiments 1 to 11, wherein the effluent from the first reactor and the effluent from the second reactor each contains 16 to 56 wt.% MTBE, and 0 to 4 wt.% isobutene. Embodiment 13 is the method of any of embodiments 1 to 12, wherein the effluent from the third reactor contains 0 to 1 wt.% isobutylene. Embodiment 14 is the method of any of embodiments 1 to 13, wherein the third reactor is operated at an operating temperature in a range 40 to 65 °C. Embodiment 15 is the method of any of embodiments 1 to 14, wherein the third reactor is operated at an operating pressure in a range of 6 to 10 bar. Embodiment 16 is the method of any of embodiments of 1 to 15, wherein MTBE in the first portion of the combined reactor effluent stream flowed in the third reactor, and/or MTBE in the third portion of the combined effluent stream flowed into the first reactor and the second reactor is capable of improving isobutylene conversion rate for MTBE synthesis.

Embodiment 17 is a method of producing methyl tertiary butyl ether (MTBE). The method includes mixing a crude C₄ stream containing isobutylene with methanol to form a feed stream and splitting the feed stream into a first feed stream and a second feed stream. The method further includes feeding the first feed stream to a first adiabatic fixed bed reactor and feeding the second feed stream to a second adiabatic fixed bed reactor. The method still further includes subjecting the isobutylene and the methanol, in the first adiabatic fixed bed reactor and the second adiabatic fixed bed reactor, respectively, to reaction conditions sufficient to cause the isobutylene to react with the methanol to produce a first portion of MTBE in effluent from the first adiabatic fixed bed reactor and in effluent from the second adiabatic fixed bed reactor. The method also includes combining effluent from the first adiabatic fixed bed reactor and effluent from the second adiabatic fixed bed reactor to form a combined reactor effluent stream, wherein the combined reactor effluent stream further contains isobutylene. In addition, the method includes reacting the isobutylene contained in a first portion of the combined reactor effluent stream with methanol in a third adiabatic fixed bed reactor that is in series with the first adiabatic fixed bed reactor and second adiabatic fixed bed reactor, to produce a third adiabatic fixed bed reactor effluent stream containing a second portion of MTBE. The method yet further includes mixing a second portion of the combined reactor effluent stream with the third adiabatic fixed bed reactor effluent stream to form a mixed intermediate product stream, recycling a third portion of the combined effluent stream to the first adiabatic fixed bed reactor and the second adiabatic fixed bed reactor, and separating the mixed intermediate product stream to form a stream containing primarily MTBE, a stream containing primarily methanol, and a C₄ raffinate stream.

Embodiment 18 is a method of producing methyl tertiary butyl ether (MTBE). The method includes mixing a crude C₄ stream containing isobutylene with methanol to form a feed stream and splitting the feed stream into a first feed stream and a second feed stream. The method further includes feeding the first feed stream to a first adiabatic fixed bed reactor and feeding the second feed stream to a second adiabatic fixed bed reactor, and subjecting the isobutylene and the methanol, in the first adiabatic fixed bed reactor and the second adiabatic fixed bed reactor, respectively, to reaction conditions sufficient to cause the isobutylene to react with the methanol to produce a first portion of MTBE in effluent from the first adiabatic fixed bed reactor and in effluent from the second adiabatic fixed bed reactor. The method still further includes combining effluent from the first adiabatic fixed bed reactor and effluent from the second adiabatic fixed bed reactor to form a stream containing MTBE, water, and isobutylene, separating water from the stream containing MTBE, water, and isobutylene to form a combined reactor effluent stream. The method also includes reacting the isobutylene contained in a first portion of the combined reactor effluent stream with methanol in a third adiabatic fixed bed reactor that is in series with the first adiabatic fixed bed reactor and second adiabatic fixed bed reactor, to produce a third adiabatic fixed bed reactor effluent stream containing a second portion of MTBE. In addition, the method includes mixing a second portion of the combined reactor effluent stream with the third adiabatic fixed bed reactor effluent stream to form a mixed intermediate product stream, recycling a third portion of the combined effluent stream to the first adiabatic fixed bed reactor and the second adiabatic fixed bed reactor, and separating the mixed intermediate product stream to form a stream containing primarily MTBE, a stream containing primarily methanol, and a C₄ raffinate stream.

## Claims

1. A method of producing methyl tertiary butyl ether (MTBE), the method comprising:
feeding isobutylene and methanol to a first reactor and a second reactor, arranged in parallel;
subjecting the isobutylene and the methanol, in the first reactor and the second reactor, respectively, to reaction conditions sufficient to cause the isobutylene to react with the methanol to produce a first portion of MTBE in effluent from the first reactor and in effluent from the second reactor;
combining effluent from the first reactor and effluent from the second reactor to form a combined reactor effluent stream, wherein the combined reactor effluent stream further comprises isobutylene;
reacting the isobutylene comprised in a first portion of the combined reactor effluent stream with methanol in a third reactor that is in series with the first reactor and second reactor, to produce a third reactor effluent stream comprising a second portion of MTBE;
mixing a second portion of the combined reactor effluent stream with the third reactor effluent stream to form a mixed intermediate product stream; [[and]]
recycling a third portion of the combined effluent stream to the first reactor and the second reactor; and
separating the mixed intermediate product stream to form a product stream comprising primarily MTBE, a stream comprising primarily methanol, and a C4 raffinate stream;
wherein the step of feeding isobutylene and methanol to the first reactor and the second reactor comprises:
mixing a crude C4 stream comprising isobutylene with methanol to form a feed stream;
splitting the feed stream into a first feed stream and a second feed stream; and
feeding the first feed stream to the first reactor and feeding the second feed stream to the second reactor;
wherein the method does not include a separation step that utilizes super fractionator column or catalytic distillation column.

2. The method of claim 1, wherein the first reactor, the second reactor, and/or the third reactor each individually include an adiabatic fixed bed reactor.

3. The method of any of claims 1 and 2, wherein the first reactor includes an adiabatic fixed bed reactor.

4. The method of any of claims 1 to 3, wherein the first reactor effluent stream and the second reactor effluent stream further comprise[[s]] water, and the combining step comprises: combining the effluent from the first reactor and the effluent from the second reactor to form a stream comprising MTBE, water, isobutylene; separating water from the stream comprising MTBE, water, isobutylene to form the combined reactor effluent stream.

5. The method of any of claims 1 to 4, wherein the first reactor and/or the second reactor each individually include a down flow reactor.

6. The method of any of claims 1 to 5, wherein the wherein the first reactor and the second reactor each comprise a catalyst that comprises a polystyrene based resin.

7. The method of any of claims 1 to 6, wherein the product stream comprises at least 98 wt.% MTBE.

8. The method of any of claims 1 to 7, wherein the third reactor is operated at a higher pressure than the first reactor and the second reactor.

9. The method of any of claims 1 to 5 and 7 to 8, wherein the first reactor and the second reactor each comprise a catalyst that comprises polystyrene based resin, polystyrene divinyl benzene based resin, sulfonic resin, macroreticular resin, acidic ion-exchange resin, sulphonated macroporous resin, or any combination thereof.

10. The method of any of claims 1 to 9, wherein the first reactor and the second reactor are each operated at an operating temperature in a range 40 to 60 °C.

11. The method of any of claims 1 to 10, wherein the first reactor and the second reactor are each operated at an operating pressure in a range of 6 to 10 bar.

12. The method of any of claims 1 to 11, wherein the effluent from the first reactor and the effluent from the second reactor each comprises 16 to 56 wt.% MTBE, and 0 to 4 wt.% isobutene.

13. The method of any of claims 1 to 12, wherein the effluent from the third reactor comprises 0 to 1 wt.% isobutylene.

14. The method of any of claims 1 to 13, wherein MTBE in the third portion of the combined effluent stream flowed into the first reactor and the second reactor is capable of improving isobutylene conversion rate for MTBE synthesis.

15. The method of any of claims 1 to 14, wherein the first reactor and the second reactor each comprise a catalyst that comprises a polystyrene divinyl benzene based resin.

## Patentansprüche

1. Verfahren zum Produzieren von Methyl-tert-butylether (MTBE), das Verfahren umfassend:
Zuführen von Isobutylen und Methanol zu einem ersten Reaktor und einem zweiten Reaktor, die parallel angeordnet sind;
Aussetzen des Isobutylens und des Methanols in dem ersten beziehungsweise dem zweiten Reaktor Reaktionsbedingungen, die ausreichen, um das Isobutylen zu veranlassen, mit dem Methanol zu reagieren, um einen ersten Anteil MTBE in Abwasser aus dem ersten Reaktor und in Abwasser aus dem zweiten Reaktor zu produzieren;
Vereinigen des Abwassers aus dem ersten Reaktor und des Abwassers aus dem zweiten Reaktor, um einen vereinigten Reaktorabwasserstrom auszubilden, wobei der vereinigte Reaktorabwasserstrom ferner Isobutylen umfasst;
Reagieren des Isobutylens, das in einem ersten Anteil des vereinigten Reaktorabwasserstroms enthalten ist, mit Methanol in einem dritten Reaktor, der mit dem ersten Reaktor und dem zweiten Reaktor in Reihe geschaltet ist, um einen dritten Reaktorabwasserstrom zu produzieren, umfassend einen zweiten Anteil MTBE;
Mischen eines zweiten Anteils des vereinigten Reaktorabwasserstroms mit dem dritten Reaktorabwasserstrom, um einen gemischten Zwischenproduktstrom auszubilden; [[und]]
Rückführen eines dritten Anteils des vereinigten Abwasserstroms zu dem ersten Reaktor und dem zweiten Reaktor; und
Trennen des gemischten Zwischenproduktstroms, um einen Produktstrom auszubilden, umfassend hauptsächlich MTBE, einen Strom, umfassend hauptsächlich Methanol, und einen C4-Raffinatstrom;
wobei der Schritt des Zuführens von Isobutylen und Methanol zu dem ersten Reaktor und zu dem zweiten Reaktor umfasst:
Mischen eines C4-Rohstroms, umfassend Isobutylen, mit Methanol, um einem Zufuhrstrom auszubilden;
Aufteilen des Zufuhrstroms in einen ersten Zufuhrstrom und einen zweiten Zufuhrstrom; und
Zuführen des ersten Zufuhrstroms zu dem ersten Reaktor und Zuführen des zweiten Zufuhrstroms zu dem zweiten Reaktor;
wobei das Verfahren keinen Trennschritt einschließt, der eine Superfraktionierkolonne oder eine katalytische Destillationskolonne nutzt.

2. Verfahren nach Anspruch 1, wobei der erste Reaktor, der zweite Reaktor und/oder der dritte Reaktor jeweils einzeln einen adiabatischen Festbettreaktor einschließen.

3. Verfahren nach einem der Ansprüche 1 und 2, wobei der erste Reaktor einen adiabatischen Festbettreaktor einschließt.

4. Verfahren nach einem der Ansprüche 1 bis 3, wobei der Abwasserstrom des ersten Reaktors und der Abwasserstrom des zweiten Reaktors ferner Wasser umfassen, und der Vereinigungsschritt umfasst: Vereinigen des Abwassers aus dem ersten Reaktor und des Abwassers aus dem zweiten Reaktor, um einen Strom auszubilden, umfassend MTBE, Wasser, Isobutylen; Trennen von Wasser aus dem Strom, umfassend MTBE, Wasser und Isobutylen, um den vereinigten Reaktorabwasserstrom auszubilden.

5. Verfahren nach einem der Ansprüche 1 bis 4, wobei der erste Reaktor und/oder der zweite Reaktor jeweils einzeln einen Abwärtsströmungsreaktor einschließen.

6. Verfahren nach einem der Ansprüche 1 bis 5, wobei der wobei der erste Reaktor und der zweite Reaktor jeweils einen Katalysator umfassen, der ein Harz auf Polystyrolbasis umfasst.

7. Verfahren nach einem der Ansprüche 1 bis 6, wobei der Produktstrom mindestens 98 Gew.-% MTBE umfasst.

8. Verfahren nach einem der Ansprüche 1 bis 7, wobei der dritte Reaktor bei einem höheren Druck als der erste Reaktor und der zweite Reaktor betrieben wird.

9. Verfahren nach einem der Ansprüche 1 bis 5 und 7 bis 8, wobei der erste Reaktor und der zweite Reaktor jeweils einen Katalysator umfassen, der Harz auf Polystyrolbasis, Harz auf Polystyroldivinylbenzolbasis, Sulfonharz, makroretikuläres Harz, saures Ionenaustauscherharz, sulfoniertes makroporöses Harz oder eine beliebige Kombination davon umfasst.

10. Verfahren nach einem der Ansprüche 1 bis 9, wobei der erste Reaktor und der zweite Reaktor jeweils bei einer Betriebstemperatur in dem Bereich von 40 bis 60 °C betrieben werden.

11. Verfahren nach einem der Ansprüche 1 bis 10, wobei der erste Reaktor und der zweite Reaktor jeweils bei einem Betriebsdruck in einem Bereich von 6 bis 10 bar betrieben werden.

12. Verfahren nach einem der Ansprüche 1 bis 11, wobei das Abwasser aus dem ersten Reaktor und das Abwasser aus dem zweiten Reaktor jeweils 16 bis 56 Gew.-% MTBE und 0 bis 4 Gew.-% Isobuten umfassen.

13. Verfahren nach einem der Ansprüche 1 bis 12, wobei das Abwasser aus dem dritten Reaktor 0 bis 1 Gew.-% Isobutylen umfasst.

14. Verfahren nach einem der Ansprüche 1 bis 13, wobei MTBE in dem dritten Anteil des vereinigten Abwasserstroms, der in den ersten Reaktor und den zweiten Reaktor strömt, in der Lage ist, die Isobutylenumwandlungsrate für die MTBE-Synthese zu verbessern.

15. Verfahren nach einem der Ansprüche 1 bis 14, wobei der erste Reaktor und der zweite Reaktor jeweils einen Katalysator umfassen, der ein Harz auf Polystyroldivinylbenzolbasis umfasst.

## Revendications

1. Procédé de production de méthyl tert-butyl éther (MTBE), le procédé comprenant:
l'introduction d'isobutylène et de méthanol dans un premier réacteur et un deuxième réacteur, disposés en parallèle;
le fait de soumettre l'isobutylène et le méthanol, dans le premier réacteur et le deuxième réacteur, respectivement, à des conditions de réaction suffisantes pour amener l'isobutylène à réagir avec le méthanol afin de produise une première partie de MTBE en effluent du premier réacteur et en effluent du deuxième réacteur;
la combinaison d'effluent du premier réacteur et d'effluent du deuxième réacteur pour former un courant d'effluent de réacteur combiné, dans lequel le courant d'effluent de réacteur combiné comprend en outre de l'isobutylène;
la réaction de l'isobutylène compris dans une première partie du courant d'effluent de réacteur combiné avec du méthanol dans un troisième réacteur qui est en série avec le premier réacteur et le deuxième réacteur, pour produire un troisième courant d'effluent de réacteur comprenant une deuxième partie de MTBE;
le mélange d'une deuxième partie du courant d'effluent de réacteur combiné avec le troisième courant d'effluent de réacteur pour former un courant de produit intermédiaire mélangé; [[et]]
le recyclage d'une troisième partie du courant d'effluent combiné dans le premier réacteur et le deuxième réacteur; et
la séparation du courant de produit intermédiaire mélangé pour former un courant de produit comprenant principalement du MTBE, un courant comprenant principalement du méthanol, et un courant de raffinat en C4;
dans lequel l'étape d'introduction d'isobutylène et de méthanol dans le premier réacteur et le deuxième réacteur comprend:
le mélange d'un courant en C4 brut comprenant de l'isobutylène avec du méthanol pour former un courant d'alimentation;
la division du courant d'alimentation en un premier courant d'alimentation et un second courant d'alimentation; et
l'introduction du premier courant d'alimentation dans le premier réacteur et l'introduction du second courant d'alimentation dans le deuxième réacteur;
dans lequel le procédé ne comporte pas d'étape de séparation qui utilise une colonne de super fractionnement ou une colonne de distillation catalytique.

2. Procédé selon la revendication 1, dans lequel le premier réacteur, le deuxième réacteur, et/ou le troisième réacteur comportent chacun individuellement un réacteur à lit fixe adiabatique.

3. Procédé selon l'une quelconque des revendications 1 et 2, dans lequel le premier réacteur comporte un réacteur à lit fixe adiabatique.

4. Procédé selon l'une quelconque des revendications 1 à 3, dans lequel le premier courant d'effluent de réacteur et le deuxième courant d'effluent de réacteur comprennent en outre de l'eau, et l'étape de combinaison comprend: la combinaison de l'effluent du premier réacteur et de l'effluent du deuxième réacteur pour former un courant comprenant du MTBE, de l'eau, de l'isobutylène; la séparation de l'eau du courant comprenant du MTBE, de l'eau, de l'isobutylène pour former le courant d'effluent de réacteur combiné.

5. Procédé selon l'une quelconque des revendications 1 à 4, dans lequel le premier réacteur et/ou le deuxième réacteur comportent chacun individuellement un réacteur à flux descendant.

6. Procédé selon l'une quelconque des revendications 1 à 5, dans lequel le dans lequel le premier réacteur et le deuxième réacteur comprennent chacun un catalyseur qui comprend une résine à base de polystyrène.

7. Procédé selon l'une quelconque des revendications 1 à 6, dans lequel le courant de produit comprend au moins 98 % en poids de MTBE.

8. Procédé selon l'une quelconque des revendications 1 à 7, dans lequel le troisième réacteur fonctionne à une pression plus élevée que le premier réacteur et le deuxième réacteur.

9. Procédé selon l'une quelconque des revendications 1 à 5 et 7 à 8, dans lequel le premier réacteur et le deuxième réacteur comprennent chacun un catalyseur qui comprend une résine à base de polystyrène, une résine à base de polystyrène divinylbenzène, une résine sulfonique, une résine macroréticulaire, une résine acide échangeuse d'ions, une résine macroporeuse sulfonée, ou toute combinaison de celles-ci.

10. Procédé selon l'une quelconque des revendications 1 à 9, dans lequel le premier réacteur et le deuxième réacteur fonctionnent chacun à une température de fonctionnement comprise entre dans une plage de 40 à 60 °C.

11. Procédé selon l'une quelconque des revendications 1 à 10, dans lequel le premier réacteur et le deuxième réacteur fonctionnent chacun à une pression de fonctionnement comprise dans une plage de 6 à 10 bars.

12. Procédé selon l'une quelconque des revendications 1 à 11, dans lequel l'effluent du premier réacteur et l'effluent du deuxième réacteur comprennent chacun de 16 à 56 % en poids de MTBE, et de 0 à 4 % en poids d'isobutène.

13. Procédé selon l'une quelconque des revendications 1 à 12, dans lequel l'effluent du troisième réacteur comprend de 0 à 1 % en poids d'isobutylène.

14. Procédé selon l'une quelconque des revendications 1 à 13, dans lequel le MTBE dans la troisième partie du courant d'effluent combiné circulant dans le premier réacteur et le deuxième réacteur est capable d'améliorer le taux de conversion de l'isobutylène pour la synthèse de MTBE.

15. Procédé selon l'une quelconque des revendications 1 à 14, dans lequel le premier réacteur et le deuxième réacteur comprennent chacun un catalyseur qui comprend une résine à base de polystyrène divinylbenzène.
